# EUROPEAN PATENT SPECIFICATION

(11) **EP 2 475 302 B1**
(45) Date of publication and mention of the grant of the patent: **28.02.2018**
(21) Application number: 10814800.8
(22) Date of filing: 07.09.2010
(51) Int. Cl.: A61B 5/113, A61B 5/08

(54) **RESPIRATORY INDUCTIVE PLETHYSMOGRAPHY BAND**
BAND FÜR RESPIRATORISCHE INDUKTIVE PLETHYSMOGRAFIE
BANDE DE PLÉTHYSMOGRAPHIE INDUCTIVE RESPIRATOIRE

(30) Priority: 11.09.2009 AU 2009904393
(43) Date of publication of application: 18.07.2012
(73) Proprietor: Compumedics Medical Innovation Pty Ltd, Abbotsford, Victoria 3067 (AU); Compumedics Germany GmbH, 78224 Singen (DE)
(72) Inventor: ZIV, Hedi, Abbotsford, Victoria 3067 (AU); BURTON, David, Abbotsford, Victoria 3067 (AU)
(74) Representative: Handsome I.P. Ltd
(86) International application number: PCT/AU2010/001149
(87) International publication number: WO 2011/029136

(56) References cited:
- US-A- 4 308 872
- US-A- 5 301 678
- US-A- 5 331 968
- US-A1- 2007 293 781
- US-A1- 2008 300 503
- US-B1- 6 359 449
- US-B1- 6 461 307

## Description

The present invention relates to the field of respiration monitoring. In particular, the present invention is directed to a respiratory inductive plethysmography band for determining the lung volume and rate of respiration of animals.

There are many uses for information related to the breathing cycles and volumes of animals. Measurement of breathing cycles and volumes may be done in a number of ways. The field of plethysmography uses changes in chest and/or abdominal volume to estimate breathing or respiratory parameters. The field of respiratory plethysmography (RP) has developed around the methods of elastomeric plethysmography, impedance plethysmography, and inductive plethysmography. Respiratory inductive plethysmography (RIP) uses an inductive band encircling the chest or abdomen as means to measure changes in chest or abdominal volume. Respiratory inductive plesthysmography exploits the principle that a current applied through a loop of wire generates a magnetic field normal to the orientation of the loop and that a change in the area enclosed by the loop creates an opposing current within the loop directly proportional to the change in the area. The movement during breathing changes the cross-sectional area of the portion of the body encircled by an RIP band, and thus changes the shape of the magnetic field generated by the band to induce an opposing current signal that can be processed and measured with an associated signal-processing unit.

In some types of RIP bands known in the art the interface between the signal-processing unit and the RIP band may be part of a LC oscillator in which the signal frequency is modulated by changes in the band inductance due to cross section area changes caused by respiratory effort. By FM demodulating the oscillator signal, it is possible to obtain a signal proportional to respiratory effort. Inductive vests and other transducers for acquiring signals representative of breathing patterns and volume and converting the signals to quantifiable forms are also known. Some bands are designed to fully encircle a subject around the thorax and/or abdomen, and others partially encircle the subject.

In particular, US6461307B1 discloses a 'variable planar area' defined by a lead extending from a first connector output to a monitoring apparatus, and a similar lead extending from a second connector output to the monitoring apparatus. That is, there are two distinctly separate and spaced connection outputs, each for one of the ends of a conductor strip. Specifically, the first connector portion is engaged with a piercing assembly and the second connector portion also engages with a piercing assembly. The connector outputs for the separate ends of the conductor strip are therefore spaced, making it possible to create a 'variable planar area' and associated electrical noise that the present invention avoids. US5331968A discloses free ends of a conducting wire extending from a substrate of a plethysmograph.

A need exists for a more sensitive RIP band than current bands that is readily adjustable and compact for comfort and ease of use. Such an RIP band would enable more accurate readings irrespective of subject size, particularly for paediatric applications. In this document, a reference to a "band" is a reference to a respiratory inductive plethysmography band.
Figure 1 shows a common use of a prior art RIP band as used for estimation of respiration volume and rate.
Figure 2 shows a diagram describing the planar area defined by a prior art RIP band.
Figure 3 shows a prior art RIP band.
Figure 4 shows an embodiment of an extensible RIP band incorporating intermeshed conducting wire.
Figure 5 shows an embodiment of an RIP band and buckle assembly in engaged position.
Figure 6 shows an embodiment the male connector side of a buckle for an RIP band.
Figure 7 shows an embodiment of the female side of a buckle for an RIP band.
Figure 8 shows the embodiment of an RIP buckle assembly of Figure 5 in disengaged state.
Figure 9 shows an embodiment of an RIP band and a hook and eyelet assembly in disengaged position.
Figure 10 shows an embodiment of an RIP band and a snap assembly in disengaged position.

It is an object of the invention to provide improved apparatus and methods for indirectly measuring the expansion and contraction of a subject. It is a further object of the invention to provide methods and apparatus for determining the volume and rate of respiration of a subject.

The invention operates to provide improved breathing movement monitoring sensitivity of a subject by incorporating an active measurement function corresponding to the full circumference of a band. The invention avoids the use of band extensions of the prior art which do not generate active inductive changes as these regions of the bands tend to desensitise the overall subject breathing detection capability for the bands.

In one aspect, the invention provides apparatus for measuring changes in the circumference of a subject, in order for a front-end unit to determine the volume and rate of respiration of the subject, the apparatus comprising: an extensible band (1) for location around the circumference of a subject; an intermeshed energisable conducting wire (10), the wire (10) having a first end and a second end; and engagement means for securing the band (1) around the subject; characterized in that: the engagement means comprises a conducting connector (7); the apparatus further comprises: a single electrical connector (5) disposed on the band, the single electrical connector (5) having a first pin and a second pin, and a cable incorporating multiple wires, the cable connected to the single electrical connector (5), the cable for transferring signals to a front-end unit; the first end of the wire (10) being in electrical communication with the first pin of the electrical connector (5); and the second end of the wire (10) being in electrical communication with the second pin of the electrical connector (5), via the conducting connector (7) to form a gapless conducting loop.

Preferably, the engagement means comprises of male and female members. More preferably, the engagement means is a buckle. However, the engagement means may be any other suitable structure used for fastening. Preferably, the engagement means comprises of metallic material. Preferably, the engagement means includes a resilient connector. Preferably an embodiment of the invention further comprises of any one or a combination of means for signal preparation, amplification, digitising, processing, transferring or wirelessly transmitting, which is preferably incorporated into the engagement means. The invention includes embodiments which may further comprise of a wireless interface for transmitting signals.

In another aspect, the invention provides a method of measuring the respiratory effort of an animal comprising of the steps of: providing the apparatus according to the first aspect; encircling the thorax and/or abdomen of the animal with the band; securing the band around the subject with the engagement means; energising the conducting wire with current; acquiring signals according to the movement of the encircling conducting wire; transferring signals to a front-end unit with the cable; and converting the signals to a measurement of respiratory effort with the front-end unit. The animal is preferably a human. The human may be an adult or a child. Alternatively, the method may be practised on other species such as horses or dogs.

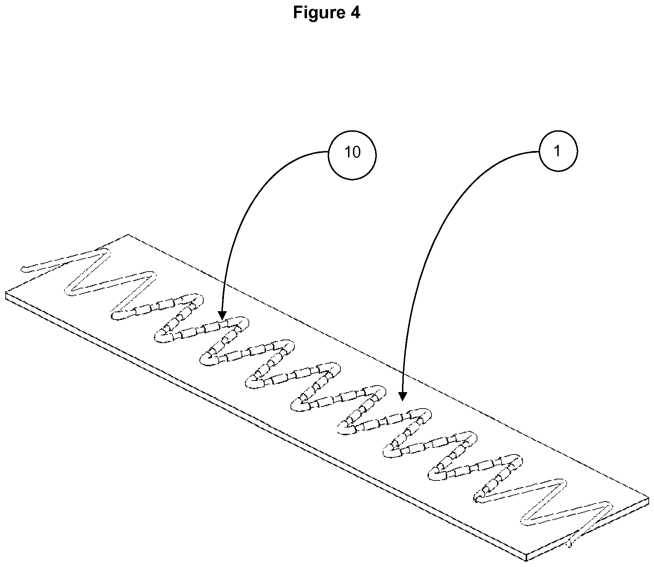

Figures 4 to 10 illustrate embodiments of the invention. It will be understood that there are many other possible embodiments of the invention and that the invention is limited only by the scope of the claims appended hereto.

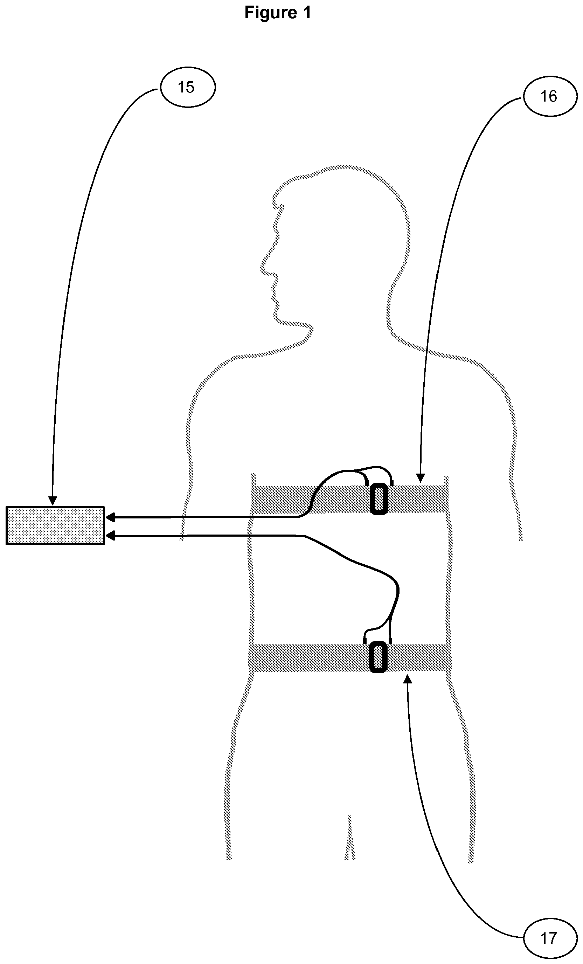

Figure 1 shows a common use of known RIP bands as used for estimation of respiration volume and rate. Two bands are usually used and placed on the abdomen (17) and thoracic region (16) of a subject. The bands are connected to a front-end unit (15), which may include impedance matching circuits, and which may communicate via replaceable or rechargeable battery-powered wireless or wired means to a processing unit, the processing unit analysing/processing the signals and obtaining a signal proportional to the respiratory effort.

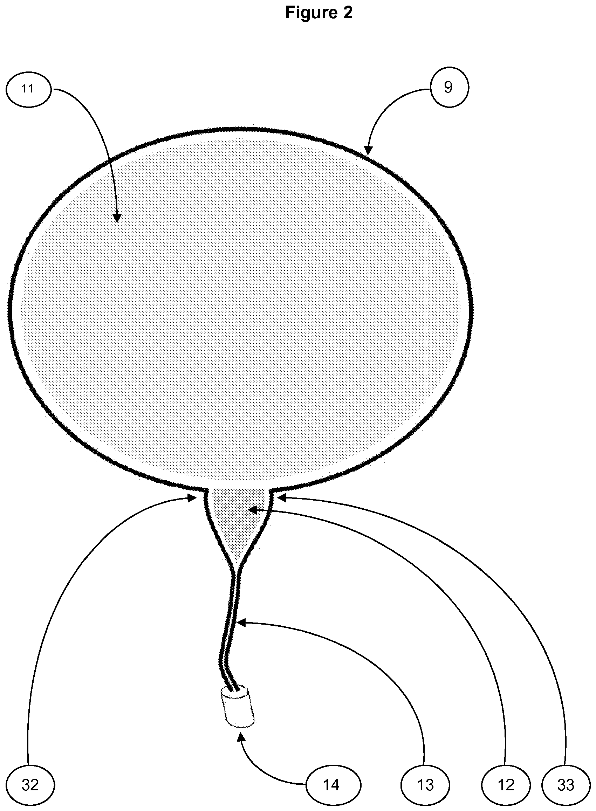

Figure 2 shows a diagram describing the generally rounded variable planar area (11), (12) defined by an RIP band (9), the band becoming the main part of a magnetic loop when energised with electrical current. The relevant total area defined by the magnetic loop includes the following sections: the main loop encircling the subject (11), the variable planar area (12) between the wires connected to the band (32 and 33) and the point the wires are moulded together (13), the small constant area between the wires in the portion of cable where the wires are moulded together (13) up to the connector (14) that may also comprise of the front end. Further, the variable planar area (12) may be disposed in the same plane or another plane from the generally rounded variable planar area (11). In this document the subject may be a human or any other animal. It will be understood that the invention will be advantageous for the monitoring of respiration in horses and other veterinary applications.

The movement during breathing changes the planar area defined by an RIP band, and thus changes the properties of the magnetic field generated by the energised band, which induces a current opposing that in the energised band, the opposing current being measureable. The movement may also affect the orientation of variable planar area and the relationship between the two planar areas (11 and 12). The variable measured current is processed to give a signal proportional to the respiratory effort causing the variations in current. Any changes in the planar area and consequent changes in the properties of the magnetic loop that are a result of the subject's breathing movements contribute to the relevant portion of the signal of interest, whereas any changes in the planar area and resultant magnetic properties changes that result from other movements contribute to inaccuracy or noise to the signal of interest. The present invention most advantageously seeks to minimise the portion of the variable planar area not contributing to the signal that is proportional to the respiration effort relative to variable planar area that contributes to the signal by fully encircling the subject, thereby reducing the noise from the variable area (12) and consequently improving the signal-to-noise ratio for the signal of interest.

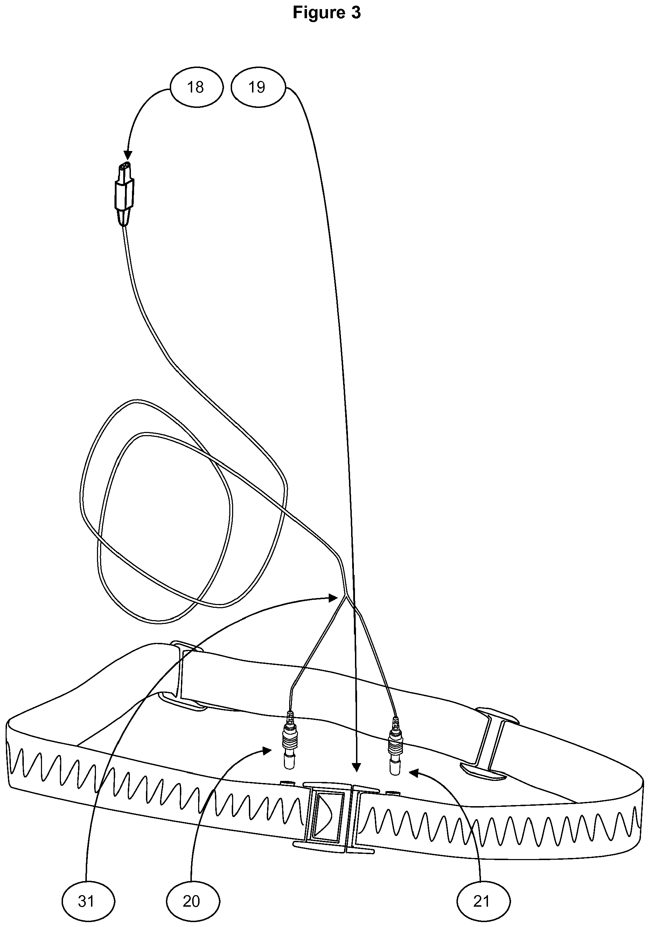

The advantage of the present invention is more easily seen by reference to Figure 3, which shows a further characteristic example of RIP band prior art. In this characteristic example, the band cross-sectional area does not fully encircle the subject because the buckle introduces a gap (19) in the complete loop. In this example, the variable planar area comprises of the cross-sectional area defined by the cable connection points to the band (20 and 21) and the point where the two wires are moulded together (31). The remainder of the magnetic loop is within the moulded cable, up to the connector (18). The variable planar area of this example in comparison with that of that of the invention illustrated in Figure 2 (12) results in relatively large extraneous signals being introduced into the respiratory signal and a resultant undesirable small ratio of signal-to-noise.

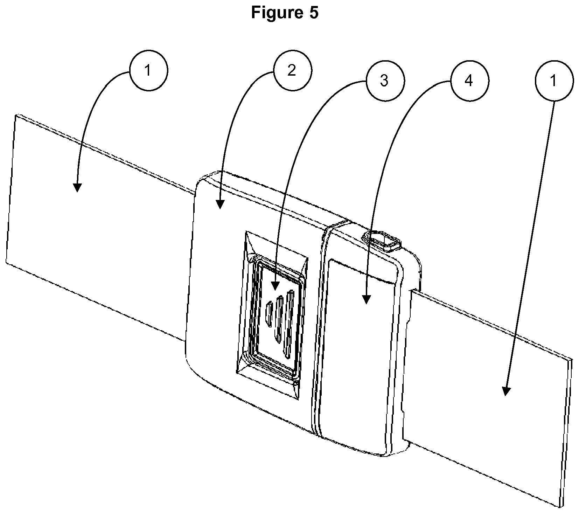

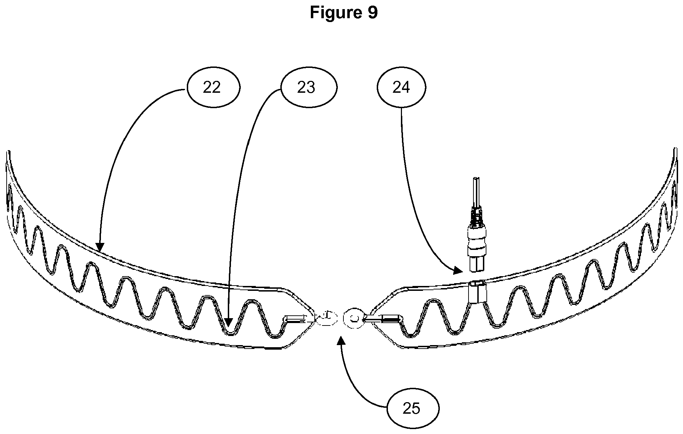

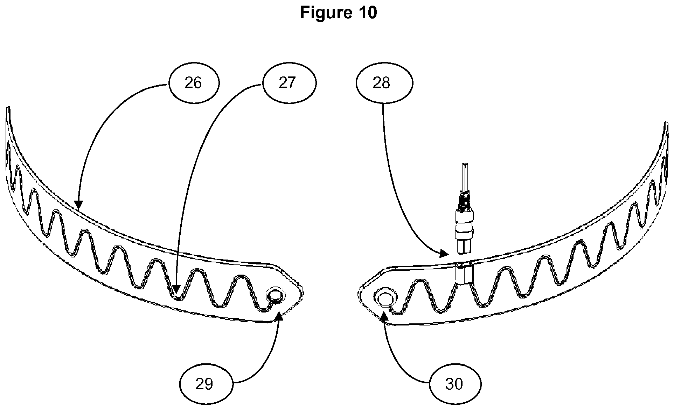

Figure 4 shows an embodiment of the invention as an RIP band (1) with an intermeshed conducting wire (10). The material of the band may be any suitable extensible material, such as a cloth incorporating elastic threads. This band is used in conjunction with an engagement means for creating a RIP band when operatively engaged. The band must fit snugly around the circumference of the subject when fitted for use. Preferably, the engagement means is a buckle as shown in Figures 5 to 8. However, other engagement means known in the art may be used. The conducting wire may or may not be intermeshed with the band. Other embodiments of the conducting wire may incorporate the wire through attachment to the band with suitable attachment means known in the art, such as loops of the mesh, pins, or the like. Also, the Figure shows the conducting wire in a "zig-zag" pattern but other patterns may be used. The invention includes any type of band and associated conducting wire which are extensible. The choice of pattern of arrangement of the conducting wire (10) depends on the signal-to-noise ratio that is produced by the pattern chosen. The pattern should allow the band to be extensible and to stretch and the wire to stretch with it. The engagement means may take other forms, such as hooks or snaps, as illustrated in Figures 9 and 10.

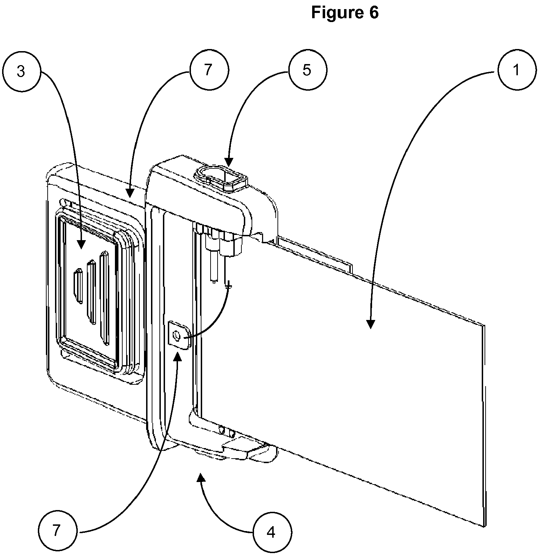

Figure 5 shows a preferred embodiment of the RIP band (1) and buckle, the band (1) forming a full circumference circuit when the buckle is engaged as shown. The end portions of the intermeshed wire (shown as 10 in Figure 4) is in electrical communication inside both the male portion of the engagement means (4) and the female portion of the engagement means (2) as described below. Preferably, the engagement means (4) incorporates a disengagement release mechanism (3) in the male portion (4) as shown in Figures 5 and 6 Figure 6 shows an embodiment of the male portion (4) of the engagement means. The band (1) intermeshed wire (see 10, Figure 4) is in electrical communication with the first pin of an electrical connector (5) to allow transferring the signals to a circuit, which may digitise the signals and transfer them to an analysis processing unit for calculating the volume and rate of respiration. Preferably, the connector comprises of metal material. However, any suitable conducting material, such as conducting polymers, may be used. The female portion of the buckle (2) is in electrical communication with the front end via the second pin of the electrical connector (5), and via conducting connector (7), when engaged with the male portion of the buckle (4). Preferably, the connector comprises of metal material. The disengagement release mechanism (3) embodiment is preferably part of the male engagement means (4), but other arrangements are possible.

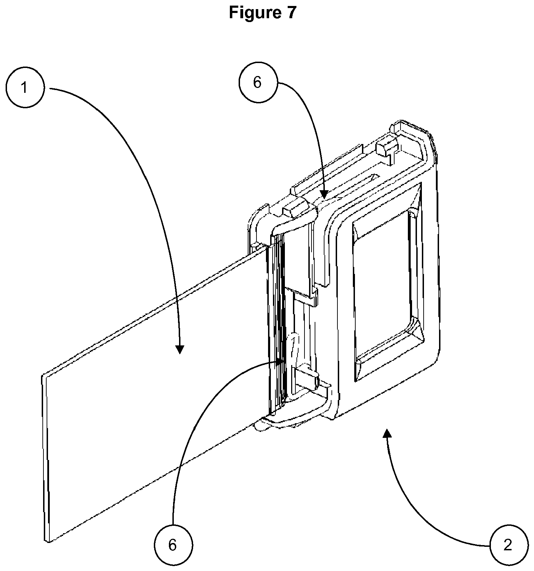

Figure 7 shows an embodiment of the female portion of the engagement means (2). The band (1) intermeshed wire (see 10, figure 4) is in electrical communication with the male portion of the engagement means (4) when engaged, via an electrical connection (6). Preferably, the electrical connection comprises of a resilient material such as in a spring.

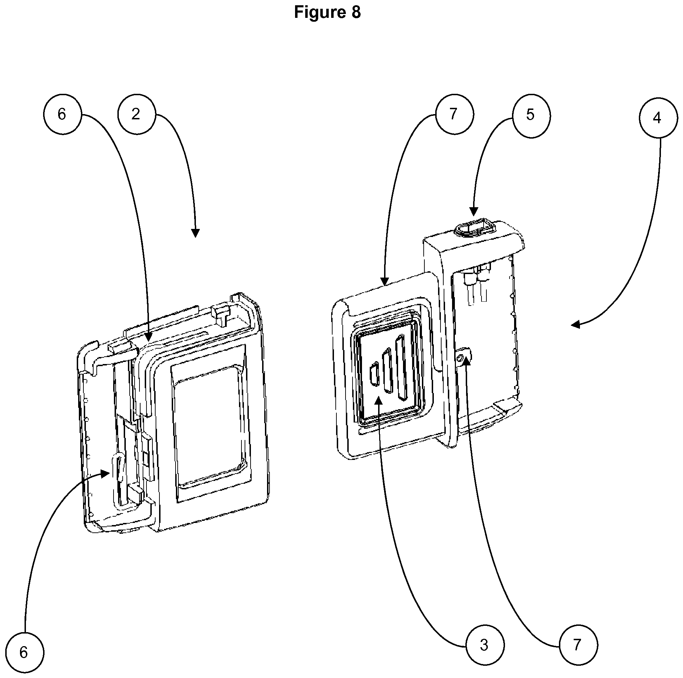

Figure 8 shows the most preferred embodiment having both male portion and female portions of the engagement means. The spring based connection (6) on the receptacle side of the buckle (2), interfaces with the metal connection interface (7) on the male end of the engagement means (4), to complete the full circumference of the circuit around the subject wearing the band. Both connections are then, in this embodiment, connected to a connector (5), transferring the signals to a circuit, which may digitise the signals and transfer them to an analysis processing unit for calculating the volume and rate of respiration. Other, less preferred, embodiments may include engagement means not having male and female portions, but which engage a connector. However such engagement means may be less effective at maintaining the circuit connection.

The invention includes multiple bands similar to those shown in Figure 1. Embodiments may incorporate impedance matching circuits, band interconnections, and which may communicate via replaceable or rechargeable battery-powered wireless or wired means to a processing unit, analysing/processing the signals and obtaining a signal proportional to respiratory effort. Embodiments having multiple bands comprise of intercommunication means for communication between bands and between bands and front-end units. A front-end unit may comprise of a connector means, a signal-acquisition means or a signal analysis means, or a combination thereof. The intercommunication means may comprise of a cable incorporating multiple wires. In such an embodiment, there may be a single electrical connector (5) such as shown in Figure 7. Other embodiments may incorporate an analysis unit within the buckle, or a replaceable or rechargeable battery-based wireless interface. Further embodiments may incorporate an impedance matching coil into the band buckle for signal improvement.

Figure 9 shows an embodiment of an RIP band and a hook and eyelet assembly in disengaged position. The band (22) serves to optimally encircle the subject. The intermeshed wire (23) serves to enable a magnetic loop area. The hook and eyelet (25) serve two means - the first to secure the band (22) around the subject and the second to enable an electric connection between both sides of the band, thus minimizing the unwanted variable cross-section area. The signals are communicated to a front-end unit via a connector (24).

Figure 10 shows an embodiment of an RIP band and a snap assembly in disengaged position. Similar to figure 9, the band (26) optimally encircles the subject. The intermeshed wire (27) enables the magnetic loop. The snap plug (29) and receptacle (30) enable an electric connection between both ends of the band and the signals are communicated onward via the connector (28).

## Claims

1. Apparatus for measuring changes in the circumference of a subject, in order for a front-end unit to determine the volume and rate of respiration of the subject, the apparatus comprising:
an extensible band (1) for location around the circumference of a subject provided with an intermeshed energisable conducting wire (10), the wire (10) having a first end and a second end; and
engagement means (4) comprising a conducting connector (7), for securing the band (1) around the subject;
**characterized in that**:
the apparatus further comprises:
a single electrical connector (5), the single electrical connector (5) having a first pin and a second pin, and
a cable incorporating multiple wires, the cable connected to the single electrical connector (5), the cable for transferring signals indicative of movement of the wire (10) to a front-end unit;
the first end of the wire (10) being in electrical communication with the first pin of the electrical connector (5); and
the second end of the wire (10) being in electrical communication with the second pin of the electrical connector (5), via an electrical connection (6) and the conducting connector (7) to form a gapless conducting loop.

2. The apparatus of claim 1, wherein the multiple wires of the cable are moulded together.

3. The apparatus of claim 1 or claim 2, wherein said engagement means comprises male and female members.

4. The apparatus of any preceding claim, wherein said engagement means is a buckle.

5. The apparatus of any preceding claim, wherein said wire (10) comprises metal material.

6. The apparatus of any preceding claim, wherein said engagement means comprises a resilient connector.

7. The apparatus of any preceding claim, further comprising signal digitising means.

8. The apparatus of claim 7, wherein the signal digitising means is incorporated into the engagement means.

9. The apparatus of any preceding claim, further comprising of a battery for providing current.

10. The apparatus of claim 9, wherein said battery is rechargeable.

11. A method of measuring the respiratory effort of an animal comprising of the steps of:
providing the apparatus according to any preceding claim;
encircling the thorax and/or abdomen of the animal with the band (1);
securing the band around the subject with the engagement means;
energising the conducting wire (10) with current;
acquiring signals according to the movement of the encircling conducting wire (10);
transferring signals to a front-end unit with the cable; and
converting the signals to a measurement of respiratory effort with the front-end unit.

12. The method of claim 11, wherein said animal is a human being.

13. The method of claim 10 or claim 11, wherein the signals are transferred to the front-end unit wirelessly.

14. The method of any one of claims 11 to 13, wherein the step of transferring is powered with a battery.

## Patentansprüche

1. Vorrichtung zur Messung von Veränderungen im Umfang eines Subjekts, um einer Front-End Einheit die Bestimmung des Volumens und der Frequenz der Atmung dieses Subjekts zu ermöglichen, wobei die Vorrichtung umfasst:
ein ausdehnbares Band (1) zur Anbringung um den Umfang eines Subjekts herum, versehen mit einem vermaschten, mit Strom versorgbaren leitenden Draht (10),
wobei der Draht (10) ein erstes Ende und ein zweites Ende aufweist; und
Eingriffsmittel (4) umfassend einen leitenden Verbinder (7), um das Band (1) um das Subjekt herum festzulegen;
**dadurch gekennzeichnet, dass**
die Vorrichtung weiterhin umfasst:
einen einzelnen elektrischen Verbinder (5), welcher einen ersten Stift und einen zweiten Stift aufweist, und ein Kabel, welches eine Vielzahl von Drähten enthält, wobei das Kabel mit dem einzelnen elektrischen Verbinder (5) verbunden ist, wobei das Kabel Signale, die eine Bewegung des Drahtes (10) anzeigen, an eine Front-End Einheit überträgt,
wobei das erste Ende des Drahtes (10) in elektrischer Verbindung steht mit dem ersten Stift des elektrischen Verbinders (5) und
wobei das zweite Ende des Drahtes (10) in elektrischer Verbindung steht mit dem zweiten Stift des elektrischen Verbinders (5), über eine elektrische Verbindung (6) und mit dem leitenden Verbinder (7), um eine lückenlose Leiterschleife zu bilden.

2. Vorrichtung nach Anspruch 1, bei der die Vielzahl von Drähten des Kabels umspritzt ist.

3. Vorrichtung nach Anspruch 1 oder 2, bei der die Eingriffsmittel männliche und weibliche Elemente umfassen.

4. Vorrichtung nach einem der vorhergehenden Ansprüche, bei der die Eingriffsmittel eine Schnalle sind.

5. Vorrichtung nach einem der vorhergehenden Ansprüche, bei der der Draht (10) ein metallisches Material umfasst.

6. Vorrichtung nach einem der vorhergehenden Ansprüche, bei der die Eingriffsmittel einen elastischen Verbinder umfassen.

7. Vorrichtung nach einem der vorhergehenden Ansprüche, weiterhin umfassend Signaldigitalisierungsmittel.

8. Vorrichtung nach Anspruch 7, bei der die Signaldigitalisierungsmittel in die Eingriffsmittel eingebunden sind.

9. Vorrichtung nach einem der vorhergehenden Ansprüche, weiterhin umfassend eine Batterie zur Bereitstellung von Strom.

10. Vorrichtung nach Anspruch 9, bei der die Batterie wieder aufladbar ist.

11. Verfahren zur Messung der Atemleistung eines Tieres umfassend die Schritte:
Bereitstellen einer Vorrichtung gemäß einem der vorhergehenden Ansprüche;
Umgeben des Thorax oder des Unterleibs des Tieres mit einem Band (1);
Sichern des Bandes um das Subjekt herum mit den Eingriffsmitteln;
Beaufschlagen des leitenden Drahtes (10) mit Strom;
Gewinnen von Signalen entsprechend der Bewegung des einfassenden leitenden Drahtes (10);
Übertragen der Signale zu einer Front-End Einheit mit dem Kabel und
Umwandeln der Signale in eine Messung der Atemleistung mittels der Front-End Einheit.

12. Verfahren nach Anspruch 11, bei dem das Tier ein menschliches Wesen ist.

13. Verfahren nach Anspruch 10 oder 11, bei dem die Signale drahtlos an die Front-End Einheit übertragen werden.

14. Verfahren nach einem der Ansprüche 11 bis 13, bei dem der Schritt des Übertragens mittels einer Batterie versorgt wird.

## Revendications

1. - Appareil pour mesurer des changements de la circonférence d'un sujet, de façon à ce qu'une unité frontale détermine le volume et la fréquence respiratoires du sujet, l'appareil comprenant :
une bande extensible (1) destinée à être placée autour de la circonférence d'un sujet comportant
un fil conducteur entremêlé pouvant être alimenté (10), le fil (10) ayant une première extrémité et une seconde extrémité ; et
un moyen de mise en prise (4) comprenant un connecteur conducteur (7), pour fixer la bande (1) autour du sujet;
**caractérisé par le fait que** :
l'appareil comprend en outre :
un connecteur électrique unique (5), le connecteur électrique unique (5) ayant une première broche et une seconde broche, et
un câble comprenant de multiples fils, le câble étant relié au connecteur électrique unique (5), le câble étant destiné à transférer des signaux indicatifs d'un déplacement du fil (10) à une unité frontale ;
la première extrémité du fil (10) étant en communication électrique avec la première broche du connecteur électrique (5) ; et
la seconde extrémité du fil (10) étant en communication électrique avec la seconde broche du connecteur électrique (5), par l'intermédiaire d'une connexion électrique (6) et du connecteur conducteur (7) pour former une boucle conductrice sans espacement.

2. - Appareil selon la revendication 1, dans lequel les multiples fils du câble sont moulés ensemble.

3. - Appareil selon la revendication 1 ou la revendication 2, dans lequel ledit moyen de mise en prise comprend des éléments mâle et femelle.

4. - Appareil selon l'une quelconque des revendications précédentes, dans lequel ledit moyen de mise en prise est une boucle.

5. - Appareil selon l'une quelconque des revendications précédentes, dans lequel ledit fil (10) comprend un matériau métallique.

6. - Appareil selon l'une quelconque des revendications précédentes, dans lequel ledit moyen de mise en prise comprend un connecteur souple.

7. - Appareil selon l'une quelconque des revendications précédentes, comprenant en outre un moyen de numérisation de signal.

8. - Appareil selon la revendication 7, dans lequel le moyen de numérisation de signal est incorporé dans le moyen de mise en prise.

9. - Appareil selon l'une quelconque des revendications précédentes, comprenant en outre une batterie pour fournir un courant.

10. - Appareil selon la revendication 9, dans lequel ladite batterie est rechargeable.

11. - Procédé de mesure de l'effort respiratoire d'un animal, comprenant les étapes consistant à :
fournir l'appareil selon l'une quelconque des revendications précédentes ;
encercler le thorax et/ou l'abdomen de l'animal avec la bande (1) ;
fixer la bande autour du sujet à l'aide du moyen de mise en prise ;
alimenter le fil conducteur (10) avec un courant ;
acquérir des signaux selon le déplacement du fil conducteur d'encerclement (10) ;
transférer des signaux à une unité frontale à l'aide du câble ; et
convertir les signaux en une mesure d'effort respiratoire à l'aide de l'unité frontale.

12. - Procédé selon la revendication 11, dans lequel ledit animal est un être humain.

13. - Procédé selon la revendication 10 ou la revendication 11, dans lequel les signaux sont transférés à l'unité frontale de manière sans fil.

14. - Procédé selon l'une quelconque des revendications 11 à 13, dans lequel l'étape de transfert est alimentée au moyen d'une batterie.
